(19) Europäisches Patentamt
European Patent Office
Office européen des brevets

(11) EP 2 719 710 B1

(12) # EUROPÄISCHE PATENTSCHRIFT

(45) Veröffentlichungstag und Bekanntmachung des Hinweises auf die Patenterteilung:
**09.03.2016 Patentblatt 2016/10**

(21) Anmeldenummer: **13187847.2**

(22) Anmeldetag: **09.10.2013**

(51) Int Cl.:
***C08C 19/06** (2006.01)* ***C08K 7/18** (2006.01)*
***C08K 7/22** (2006.01)* ***C08K 9/08** (2006.01)*
***B32B 5/30** (2006.01)* ***B32B 19/04** (2006.01)*
***B32B 25/02** (2006.01)*

(54) **Elastomerprodukt mit kovalent gebundenen Partikel**

Elastomer product with covalently bonded particles

Produit élastomère comprenant des particules liées de manière covalente

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.10.2012 AT 10872012**

(43) Veröffentlichungstag der Anmeldung:
**16.04.2014 Patentblatt 2014/16**

(73) Patentinhaber: **Semperit Aktiengesellschaft Holding**
**1031 Wien (AT)**

(72) Erfinder:
- **Holzner, Armin**
  **2630 Ternitz (AT)**
- **Kern, Wolfgang**
  **8055 Seiersberg (AT)**
- **Lenko, Dietmar**
  **8051 Graz (AT)**
- **Manhart, Jakob Cornelius**
  **8700 Leoben (AT)**
- **Schaller, Raimund**
  **2620 Neunkirchen (AT)**
- **Schlögl, Sandra**
  **8152 Stallhofen (AT)**

(74) Vertreter: **Burger, Hannes**
**Anwälte Burger & Partner**
**Rechtsanwalt GmbH**
**Rosenauerweg 16**
**4580 Windischgarsten (AT)**

(56) Entgegenhaltungen:
**DD-A5- 296 853 US-A1- 2006 074 185**

- **AMORNCHAIYAPITAK C ET AL: "Modification of epoxidised natural rubber film surface by polymerisation of methyl methacrylate", EUROPEAN POLYMER JOURNAL, PERGAMON PRESS LTD. OXFORD, GB, Bd. 44, Nr. 6, 1. Juni 2008 (2008-06-01), Seiten 1782-1788, XP022703260, ISSN: 0014-3057, DOI: 10.1016/J.EURPOLYMJ.2008.03.002 [gefunden am 2008-03-14]**
- **B. THONGNUANCHAN, K. NOKKAEW, A. KAESAMAN, C. NAKASON: "Epoxidized Natural Rubber-Bonded Para Rubber Wood Particleboard", POLYMER ENGINEERING & SCIENCE, Bd. 47, Nr. 4, 1. März 2007 (2007-03-01), - 1. März 2007 (2007-03-01), Seiten 421-428, XP002718002,**
- **SRUANGANURAK A ET AL: "Layer-by-layer assembled nanoparticles: A novel method for surface modification of natural rubber latex film", COLLOIDS AND SURFACES. A, PHYSICACHEMICAL AND ENGINEERING ASPECTS, ELSEVIER, AMSTERDAM, NL, Bd. 289, Nr. 1-3, 15. Oktober 2006 (2006-10-15), Seiten 110-117, XP027995496, ISSN: 0927-7757 [gefunden am 2006-10-15]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Anbindung von anorganischen oder organischen Feststoffpartikeln an die Oberfläche eines Elastomershandschuhs, wobei das Elastomer in der Molekülstruktur ungesättigte Kohlenstoff-Kohlenstoff Bindungen aufweist, die zumindest im Bereich der Oberfläche des Elastomerhandschuhs zumindest teilweise durch Epoxidierung abgesättigt werden, sowie einen Elastomerhandschuh aus einem Elastomer mit einer Oberfläche, wobei das Elastomer in der Molekülstruktur ungesättigte Kohlenstoff-Kohlenstoff Bindungen aufweist.

Die Modifizierung der Oberfläche von Naturkautschukhandschuhen ist aus dem Stand der Technik bereits bekannt. Beispielsweise wird die Oberfläche mit Beschichtungen versehen oder aufgeraut, um damit eine bessere Gleitfähigkeit der Handschuhe zu erreichen. Insbesondere soll damit die Anziehbarkeit bzw. die Nassanziehbarkeit der Handschuhe verbessert werden. Es sind auch Funktionalisierungen zur Reduktion des Allergiepotentials, das Naturkautschuk anhaftet, bekannt.

Zur Modifizierung der Oberfläche von Naturkautschuk ist aus dem Stand der Technik weiter die Epoxidierung der Kautschukoberfläche bekannt.

So beschreibt die US 7,442,746 A ein Verfahren zur Synthese eines epoxidierten Polymers, das die folgenden Schritte umfasst: (1) Herstellung eines kationisch stabilisierten Polymerlatex mit mindestens einem konjugierten Diolefinmonomer in einer ersten Stufe, (2) Behandeln des Polymerlatex aus Schritt (1) mit Ameisensäure oder Essigsäure und Wasserstoffperoxid, und (3) Reagieren der Mischung für eine vorbestimmte Zeit und vorbestimmte Temperatur, um den gewünschten Grad an Epoxidierung zu erreichen.

Aus der DE 102 60 219 B ist bekannt, dass es durch Eintauchen eines Kautschukproduktes in oder durch Beschichten mit einer Behandlungsflüssigkeit, die hergestellt wird durch Zugabe von Wasserstoffperoxid und anderen Zusätzen, wie Tensid, Alkohol, Verdickungsmittel, eines anderen (weiteren) Mittels zur Herabsetzung der Oberflächenspannung, zu einer wässrigen Essigsäure- oder Ameisensäure-Lösung möglich ist, die Oberflächenschicht des Kautschukprodukts zu epoxidieren, um dadurch dieser Oberfläche nicht-haftende bzw. nicht-klebrige Eigenschaften, Gleiteigenschaften und andere Sperrschicht-Eigenschaften zu verleihen, ohne dass toxische Gase auftreten und ohne dass eine Kontamination durch Pulverstaub während des Herstellungsverfahrens auftritt, und ohne dass die dem Kautschukprodukt eigenen Eigenschaften, wie Elastizität, Dehnung und Zugfestigkeit, beeinträchtigt (verschlechtert) werden.

Die GB 1396090 A beschreibt ein Verfahren zur Herstellung eines Gegenstandes, umfassend das Kontaktieren eines Alkylhypohalogenits oder durch Halogen substituiertes Alkylhypohalogenits mit einem geformten Gegenstand aus einem Gummi mit ethylenischen Doppelbindungen und Verbinden einer Beschichtung oder eines anderen Gummis, eines Metalls oder von Holz mit der behandelten Oberfläche des Formkörpers. Die Verklebung kann mit Hilfe eines Epoyxbindemittels erfolgen.

Die US 5,310,819 A beschreibt an der Oberfläche epoxidierte elastomere Gegenstände, bei denen die ethylenischen Bindungen des Elastomers durch Eintauchen der Gegenstände in eine Epoxidierungslösung für eine ausreichende Zeit gesättigt werden.

Aus der US 5,804,318 A sind verbesserte schmierende Beschichtungen zur Verringerung der Reibungskoeffizienten der Oberflächen von medizinischen Vorrichtungen bekannt. Die gleitfähigen Hydrogelbeschichtungen sind kovalent mit den epoxid-funktionalisierten Oberflächen verbunden.

Die US 6,797,783 A beschreibt einen Naturkautschuk, der durch Modifikation eines deproteinierten Naturkautschuks mit einem Stickstoffgehalt von weniger als 0,10 Gew.-% erhalten wird, wobei die Modifikation das Epoxidieren des deproteinierten Naturkautschuks mit Trifluorperessigsäure umfasst.

Die US 2006/074185 A1 beschreibt eine Tinte für den Tintenstrahldruck. Diese weist epoxidierte Latexpartikel auf. Über die Epoxidgruppen werden u.a. Farbestoffe an die Latexpartikel angebunden.

Amornchaiyapitak C. et al: "Modification of epoxidised natural rubber film surface by polymerisation of methyl methacrylate", European Polymer Journal, Pergamon Press Ltd. Oxford, GB, Bd. 44, Nr. 6, 1. Juni 2008, Seiten 1782-1788 beschreibt den Einsatz von Methylmethacrylat in der Herstellung von Naturgummihandschuhen zur Reduktion der Reibung. Dabei wird epoxidierter Naturgummilatex verwendet.

Thongnuanchan B. et al: "Epoxidized natural nubber-bonded wood Particleboard", Polymer Engineering & Science, Bd. 47, Nr. 4, 1. März 2007, Seiten 421-428 beschreibt die Herstellung von Spanplatten aus Holzteilchen, die unter Verwendung eines epoxidierten Naturgummis miteinander verbunden werden.

Aus der DD 296 853 A5 ist ein ein Verfahren zur Herstellung oberflächenfunktionalisierter Trägermaterialien bekannt, die als Trennmedien, Chromatographiematerialien, Ionenaustauscher, Festphasensynthesematerialien oder als Katalysatorträger einsetzbar sind. Die Herstellung erfolgt durch die chemische Fixierung von Gemischen aus epoxidierten Polydienen bzw. Polydiencopolymeren mit reaktiven organischen Verbindungen oder von Umsetzungsprodukten aus epoxidierten Polydienen bzw. Polydiencopolymeren mit reaktiven organischen Verbindungen wie Aminen, Carbonsäuren, Aminosäuren und deren Derivaten an der Oberfläche anorganischer Festkörper, wie pulverisierte amorphe oder keramisierte Gläser, gefällte oder pyrogene Kieselsäuren sowie Haupt- und Nebengruppenmetalloxiden.

Der vorliegenden Erfindung liegt die Aufgabe zugrunde, eine Möglichkeit zur Oberflächenmodifizierung von

Elastomerhandschuhen zu schaffen.

Gelöst wird diese Aufgabe einerseits indem bei dem eingangs genannten Verfahren die Feststoffpartikel nach der Epoxidierung kovalent an die Epoxidgruppen angebunden werden bzw. bei dem eingangs genannten Elastomerhandschuh die Oberfläche zumindest teilweise kovalent an das Elastomer gebundene anorganische oder organische Feststoffpartikel aufweist.

Von Vorteil ist dabei, dass durch die kovalent an das Elastomer angebundenen anorganische oder organische Feststoffpartikel (im Folgenden als Partikel bezeichnet) eine Verbesserung der Gleitfähigkeit der Elastomerhandschuhe (im Folgenden als Elastomerprodukte bezeichnet) erreicht wird. Die Anziehbarkeit, insbesondere die Nassanziehbarkeit, von Elastomerhandschuhen kann damit verbessert werden. Durch die kovalente Anbindung wird erreicht, dass die Eigenschaftsverbesserung bzw. -änderung durch die Oberflächenmodifikation über einen längeren Zeitraum erhalten bleibt. Darüber hinaus kann mit den Partikeln dem Elastomerprodukt eine zusätzliche Funktionalität verliehen werden, wenn die Partikel entsprechend gewählt werden, beispielsweise bei Verwendung von mit Wirkstoffen beladenen Partikeln. Die Anbindung der Partikel über Epoxidgruppen hat den Vorteil, dass die Epoxidierung der Elastomeroberfläche thermisch durchgeführt werden kann, also keine aktinische Strahlung für die Vorbereitung der Oberfläche erforderlich ist. Darüber hinaus wird durch nicht reagierte Epoxygruppen an der Oberfläche des Elastomers dessen Klebrigkeit reduziert und kann durch die zumindest teilweise Sättigung der ethylenischen Gruppen des Elastomers eine Verbesserung der Alterungsbeständigkeit erreicht werden.

Vorzugsweise sind die Partikel durch anorganische Partikel gebildet. Es kann damit eine Reduktion der Klebrigkeit bzw. eine Verbesserung der Anziehbarkeit, insbesondere der Nassanziehbarkeit, von Handschuhen erreicht werden, indem die Kontaktfläche des Elastomers mit einer Hand reduziert wird. Generell kann damit die Haftung eines Elastomerproduktes an einer Oberfläche aufgrund dieses Effektes reduziert werden. Darüber hinaus ist es damit aber auch möglich, dass über diese Feststoffpartikel eine zusätzliche Funktionalität in das Elastomerprodukt eingebracht wird, beispielsweise wenn Feuchtigkeit absorbierende Feststoffpartikel verwendet werden.

Zur besseren Anbindung der Feststoffpartikel an die funktionalisierte Oberfläche des Elastomerproduktes ist es von Vorteil, wenn die Feststoffpartikel vor der Reaktion ebenfalls oberflächlich funktionalisiert werden.

Die Funktionalisierung der Feststoffpartikel kann dabei durch Erzeugung von freien Mercaptogruppen und/oder freien Aminogruppen und/oder Carbonsäuregruppen und/oder Epoxidgruppen und/oder Hydroxygruppen und/oder Anhydridgruppen und/oder Isocyanatgruppen und/oder Isothiocyanatgruppen, an der Oberfläche der Feststoffpartikel erfolgen. Durch den Einsatz dieser funktionellen Gruppen wird der Vorteil erreicht, dass damit Ankergruppen mit hoher Reaktivität für die Anbindung der Feststoffpartikel an die Elastomeroberfläche zur Verfügung stehen.

Die Funktionalisierung der Feststoffpartikel kann aber auch mit zumindest einer chemischen Verbindung durchgeführt werden, die ausgewählt ist aus einer Gruppe umfassend oder bestehend aus Acrylatgruppen, Anhydridgruppen, Isocyanatgruppen, Isothiocyanatgruppen, Methacrylatgruppen, Vinylgruppen. Durch den Einsatz dieser funktionellen Gruppen wird der Vorteil erreicht, dass damit Ankergruppen für die Ankopplung von weiteren funktionellen Verbindungen an die Feststoffpartikel zur Verfügung gestellt werden können.

Um so genannte "puderfreie" Elastomerhandschuhe bereit zu stellen, ist nach einer anderen Ausführungsvariante vorgesehen, dass rein adhäsiv gebundene Partikel von der Oberfläche des Elastomerproduktes entfernt werden. Es kann damit das Allergiepotential der Elastomerprodukte reduziert werden. Zudem können damit diese Partikel, die im Vergleich zu den kovalent gebundenen Partikeln eine geringere Wirkung haben, gegebenenfalls in den Produktionsprozess zurückgeführt werden. Es wird damit eine Wundkontamination mit Partikeln bei Einsatz des Elastomerproduktes im Medizinbereich vermieden. Das Elastomerprodukt ist darüber hinaus für den Einsatz in Reinräumen geeignet.

Die Epoxidierung des Elastomers kann an einer festen Oberfläche des Elastomerproduktes durchgeführt werden. Diese Verfahrensvariante wird insbesondere zur Herstellung von einschichtigen Elastomerprodukten eingesetzt, da es damit möglich ist, gezielt Oberflächenbereiche des Elastomers zu modifizieren. Darüber hinaus können durch die Vermeidung der bulk-Epoxidierung die Alterungseigenschaften des Elastomers verbessert werden.

Neben dieser Ausführungsvariante des Verfahrens besteht im Rahmen der Erfindung auch die Möglichkeit, dass die Epoxidierung an einem Latex in flüssiger Phase durchgeführt wird. Diese Verfahrensvariante kann für die Herstellung mehrschichtiger Elastomerprodukte verwendet werden. Der Vorteil dabei ist, dass mit dieser Verfahrensvariante bei Bedarf die Epoxidierung nicht nur im Bereich der Oberfläche erfolgen kann sondern bereits an den einzelnen Latexpartikeln, wodurch eine Anpassung des Eigenschaftspotentials des Elastomerproduktes erfolgen kann.

Es ist weiter möglich, dass die Epoxidierung nur in diskreten Bereichen des Elastomers durchgeführt wird. Es kann damit eine stärkere Strukturierung der Elastomeroberfläche erreicht werden, wodurch die Gleitfähigkeit des Elastomers beeinflusst werden kann. Zudem können bestimmten Bereichen des Elastomers spezifische Eigenschaften verliehen werden. Vorzugsweise weisen die Partikel einen Partikeldurchmesser zwischen 10 nm und 10 $\mu$m auf. Mit Partikel unterhalb von 10 nm wird zwar noch ein Effekt beobachtet, allerdings ist dieser in Hinblick auf die Verbesserung der Gleitfähigkeit des Elastomers nur ungenügend ausgebildet. Mit Partikelgrößen von mehr als 10 $\mu$m konnte hingegen beobachtet werden, dass die Verbesserung der Anbindung an die Elastomeroberfläche aufgrund der Größe der Partikel wieder abnimmt.

Nach einer anderen Ausführungsvariante kann vorgesehen werden, dass die Partikel miteinander zumindest teilweise vernetzt werden. Dies kann beispielsweise über nicht reagierte funktionelle Gruppen an der Oberfläche der Partikel erfolgen. Es kann damit eine "netzartige" Struktur erzielt werden. Daneben kann die Haftung der Partikel an der Elastomeroberfläche verbessert werden, indem die Ortfestigkeit der Partikel durch die Vernetzung verbessert werden kann.

Die Partikel können zumindest einen Wirkstoff aufweisen, wodurch das Spektrum der Funktionalisierung der Elastomeroberfläche deutlich vergrößert werden kann. Gegebenenfalls kann eine Nachbeladung der Partikel durchgeführt werden, wodurch die Gebrauchsdauer des Elastomerproduktes verlängert werden kann.

Zum besseren Verständnis der Erfindung wird diese anhand der nachfolgenden Figur näher erläutert.

Es zeigt in schematisch vereinfachter Darstellung:

Fig. 1 die Modifizierung von NR-Latexfilmen durch Epoxidierung und nachfolgende Anbindung von aminofunktionalisierten $SiO_2$-Partikeln.

Unter einem Elastomerprodukt wird ein Handschuh, vorzugsweise ein chirurgischer Handschuh oder ein Untersuchungshandschuh verstanden.

Der Vollständigkeit halber sei angemerkt, dass unter einem Elastomerprodukt im Rahmen der Erfindung ein Produkt aus einem Elastomer verstanden wird, das in der Molekülstruktur ungesättigte Kohlenstoff-Kohlenstoff Bindungen aufweist, also insbesondere ethylenische Bindungen (=Dienkautschuk). Vorzugsweise ist das Elastomer ein Naturkautschuk oder ein synthetischer Isoprenkautschuk. Daneben ist die Erfindung auch auf andere derartige, ungesättigte Kohlenstoff-Kohlenstoff Bindungen aufweisende Kautschukarten anwendbar, insbesondere insbesondere Homopolymere und Copolymere wie Nitril-Butadien-Kautschuk, carboxylierter Nitril-Butadien-Kautschuk, Polybutadien, Polychloropren, Styrol-Butadien-Kautschuk.

Das Tauchverfahren zur Herstellung von Gummihandschuhen ist im Stand der Technik bereits ausführlich beschrieben. Üblicherweise umfasst es zumindest folgende Schritte: Bereitstellen einer Tauchform, Auftauchen eines Koagulanten, Auftauchen eines Latex. Daneben umfasst dieses Tauchverfahren diverse Wasch- und Trockenschritte. Üblicherweise wird dieses Tauchverfahren kontinuierlich durchgeführt, beispielsweise in einer so genannten Kettentauchanlage. Für weitere Einzelheiten dazu sei auf den einschlägigen Stand der Technik verwiesen.

Sämtlichen Ausführungsvarianten der Erfindung ist gemein, dass die ungesättigten Kohlenstoff-Kohlenstoff Bindung zumindest im Bereich der Oberfläche des Elastomerproduktes bzw. des Elastomers (im Folgendem wird nur mehr auf ein Elastomer Bezug genommen, wobei diese Bezugnahme auch das Elastomerprodukt einschließt) zumindest teilweise, vorzugsweise zu zumindest 2 %, insbesondere zwischen 10 % und 80 %, durch Epoxidierung abgesättigt werden.

Prinzipiell bestehen zwei Verfahrensvarianten. Zum einen ist es möglich, die Epoxidierung an einer festen Oberfläche des Elastomers durchzuführen. Zum anderen besteht die Möglichkeit, dass die Epoxidierung in der Flüssigphase des Latex erfolgt, und danach eine entsprechende Form in den Latex getaucht und damit der Elastomerartikel hergestellt wird.

Bei der Ausführungsvariante des Verfahrens an der festen Oberfläche des Elastomers ist es nicht zwingend notwendig, dass das Elastomerprodukt nach einem Tauchverfahren hergestellt wird. Es sind alle formgebenden Verfahren, die aus dem Stand der Technik bekannt sind, beispielsweise Spritzgussverfahren, Extrusionsverfahren, Compression Molding, etc. einsetzbar, wenngleich das Tauchverfahren im Rahmen der Erfindung das bevorzugte Verfahren zur Herstellung des Elastomerproduktes ist.

Für die Absättigung bzw. Reaktion der ungesättigten Kohlenstoff-Kohlenstoff Bindungen an einer festen Oberfläche eines Elastomerproduktes, beispielsweise eines Elastomerfilmes, wird die Elastomeroberfläche mit dem jeweiligen Reagens in Kontakt gebracht, beispielsweise in das Epoxidierungsreagens eingetaucht oder mit diesem besprüht. Das Elastomer wird bevorzugt vorvernetzt eingesetzt, wobei die Vorvernetzung bevorzugt photochemisch mit einem Thiol durchgeführt wird, wie dies in den Druckschriften AT 502 764 A1 und AT 508 099 A1 beschrieben ist. Generell wird im Rahmen der Erfindung die Vorvernetzung bevorzugt photochemisch mit einem Thiol durchgeführt. Es sind aber auch sämtliche anderen Arten der Vorvernetzung, beispielsweise die Schwefelvernetzung oder die peroxidische Vernetzung bzw. generell eine Vernetzung mittels aktinischer Strahlung im Rahmen der Erfindung möglich.

Ebenso kann eine Schwefelvernetzung (bei erhöhter Temperatur), wie sie prinzipiell aus dem Stand der Technik bekannt ist, als Vorvernetzung durchgeführt werden.

Das Reagens zur Epoxidierung des Elastomers kann beispielsweise eine aliphatische oder aromatische Persäure, z.B. Peressigsäure, Perameisensäure, Trifluorperessigsäure, Permaleinsäure, Perbenzoesäure, Monoperphthalsäure, o-Sulfoperbenzoesäure, p-Nitroperbenzoesäure und m-Chlorperbenzoesäure sein, wobei die erstere bevorzugt ist. Die Konzentration der Persäure kann zwischen 1 Gew.-% und 41 Gew.-%, insbesondere zwischen 2 Gew.-% und 4 Gew.-%, Rest Wasser (mit Wasserstoffperoxid und Carbonsäure(n)), betragen. Es werden handelsübliche Persäuren verwendet.

Es können aber auch andere Epoxidierungsmittel verwendet werden, wie beispielsweise Wasserstoffperoxid in saurem oder alkalischem Medium oder tert.-Butylhydroperoxid in alkalischem Medium oder kann es die Epoxidierung des Elastomers entsprechend einem der voranstehend genannten Dokumente zum Stand der Technik, auf die diesbezüglich ausdrücklich Bezug genommen wird, durchgeführt werden. Ebenso ist die Epoxidierung mit Katalysatoren möglich, z.B. Metall-Salen Komplex + NaOCl (Jacobsen-Epoxidierung) oder Shi-Katalysator + Oxon (Shi-Epoxidierung).

Es ist auch die in situ Bildung von Persäuren durch Reaktion einer Carbonsäure mit $H_2O_2$ möglich, z.B. die in situ Bildung von Perameisensäure.

Die Temperatur, bei der die Behandlung mit dem Epoxidierungsreagens durgeführt wird, kann zwischen 30 °C und 70 °C, insbesondere zwischen 40 °C und 50 °C betragen. Weiter kann die Dauer der "Benetzung" zwischen 1 Minute und 400 Minuten, insbesondere zwischen 40 Minuten und 100 Minuten, betragen. Danach kann das benetzte Elastomer, z.B. für eine Zeitspanne zwischen 30 Sekunden und 5 Minuten, in Wasser eingetaucht werden, um überschüssiges Epoxidierungsreagens abzuspülen.

Abschließend erfolgt eine Trocknung des benetzten Elastomers bei einer Temperatur zwischen 20 °C und 100 °C, insbesondere zwischen 55 °C und 70 °C. Vorzugsweise erfolgt die Trocknung innerhalb einer Zeitspanne zwischen 10 Minuten und 100 Minuten, insbesondere zwischen 15 Minuten und 30 Minuten.

Es ist auch möglich, dass die Benetzung des Elastomers mit dem Epoxidierungsreagens in mehreren Schritten erfolgt, wobei gegebenenfalls zwischen den einzelnen Schritten eine Zwischentrocknung erfolgt.

Durch die Epoxidierung werden an der Oberfläche des Elastomers die ethylenischen Doppelbindungen zumindest teilweise u.a. zu einem Oxiranring reagiert. Diese Oxiranringe stehen in der Folge für die kovalente Anbindung der Partikel an das Elastomer als funktionelle Gruppen zur Verfügung.

Nach einer anderen Verfahrensvariante ist vorgesehen, dass die Epoxidierung an einem, gegebenenfalls vorvernetzten, Latex in flüssiger Phase durchgeführt wird. Dieser Latex wird dann in der Folge zum Elastomerprodukt geformt, beispielsweise getaucht. Gegebenenfalls kann der Latex auf einen, insbesondere vorvernetzten, vorzugsweise photochemisch vorvernetzten, Latexfilm zur Herstellung eines zumindest zweischichtigen Elastomerproduktes aufgetaucht werden.

Für diese Verfahrensweise wird eine Suspension aus dem Latex hergestellt. Zur Stabilisierung kann dieser Suspension zumindest ein Stabilisierungsmittel, beispielsweise ein Polyethylenglycolnonylphenylether, z.B. Synperonic® NP 30, zugesetzt werden. Das Stabilisierungsmittel kann in Wasser gelöst und anschließend zum Latex oder dem Latex ohne weitere Wasserzugabe direkt zugegeben werden. Der Summenanteil an dem zumindest einen Stabilisierungsmittel kann zwischen 2 phr und 15 phr (parts per hundred parts rubber) betragen.

Der Latex kann einen Feststoffgehalt zwischen 20 % drc (dry rubber content) und 60 % drc aufweisen.

Der Latex kann mit einer Temperatur zwischen 20 °C und 60 °C eingesetzt werden.

In der Folge kann die Suspension angesäuert werden, wobei vorzugsweise ein pH-Wert zwischen 2 und 5, insbesondere zwischen 3 und 4, eingestellt wird. Zur pH-Werteinstellung wird vorzugsweise eine Carbonsäure, insbesondere Essigsäure, verwendet. Es können aber auch andere Säuren verwendet werden, beispielsweise anorganische Säuren, wie HCl. Dabei ist auch der Einsatz eines Puffers möglich. Als Puffersubstanzen eignen sich Natrium-, Kalium-, Calcium- oder Magnesiumformiat, -acetat oder -butyrate, Natriumdihydrogenphosphat, Dinatriumhydrogenphosphat oder Natrium-, Kalium-, Calcium- oder Magnesiumcarbonat, wobei Natriumcarbonat bevorzugt ist.

Das Epoxidierungsreagens kann dieser Suspension entweder zugesetzt werden oder in dieser in situ erzeugt werden.

Für erstere Variante wird vorzugsweise wiederum eine Persäure, insbesondere Peressigsäure, verwendet, wobei auch andere Säuren, z.B. wie voranstehend ausgeführt, verwendet werden können. Die Persäure wird insbesondere als wässrige Lösung mit einer Konzentration zwischen 1 Gew.-% und 41 Gew.-% eingesetzt.

Besonders bevorzugt wird eine Menge an Persäure zugesetzt, dass ein Verhältnis der molaren Konzentration an Persäure zur molaren Konzentration an Polyisopreneinheiten des Elastomers zwischen 0,04 und 0,7 beträgt.

Bei direkter Zugabe der Persäure kann die Konzentration zwischen 4 Mol-% und 70 Mol-%, bezogen auf die Isopreneinheiten, betragen.

Bei in-situ Herstellung der Persäure kann die Konzentration der Carbonsäure zwischen 10 Mol-% und 120 Mol-%, jene von $H_2O_2$ zwischen 10 Mol-% und 120 Mol-%, jeweils bezogen auf die Isopreneinheiten, betragen.

Für die in situ Erzeugung des Epoxidierungsreagens wird, wie voranstehend ausgeführt, der Suspension ein Oxidationsmittel, insbesondere Wasserstoffperoxid, und eine Carbonsäure, insbesondere Ameisensäure oder Essigsäure, zugesetzt. Das Oxidationsmittel und die Carbonsäure können in äquimolaren Mengen zugegeben werden.

Die Carbonsäure kann, insbesondere als wässrige Lösung, mit einer Konzentration zwischen 20 Gew.-% und 60 Gew.-% eingesetzt werden.

Das Oxidationsmittel kann, insbesondere als wässrige Lösung, mit einer Konzentration zwischen 25 Gew.-% und 50 Gew.-% eingesetzt werden.

Besonders bevorzugt wird eine Menge an Oxidationsmittel und Carbonsäure zugesetzt, dass ein Verhältnis der Konzentration der daraus erzeugten Persäure, insbesondere Perameisensäure, zur Konzentration an Polyisopreneinheiten des Elastomers zwischen 0,1 und 0,3 beträgt.

Die Epoxidierung in flüssiger Phase kann bei einer Temperatur zwischen 20 °C (Raumtemperatur) und 80 °C und/oder für eine Zeitspanne zwischen 20 Minuten und 60 Stunden erfolgen. Beendet wird die Epoxidierung der Latexpartikel durch Neutralisation, beispielsweise mit einer 10 Gew.-%igen Kaliumhydroxydlösung.

Aus dieser so vorbereiteten Suspension wird in der Folge das Elastomerprodukt hergestellt, insbesondere durch Tauchen einer Form.

Gegebenenfalls kann die Formgebung in mehreren Schritten durchgeführt werden, beispielsweise in zwei bis acht Wiederholungen.

Vor und oder nach der Epoxidierung kann der behandelte Latex noch mit weiteren Prozesschemikalien, wie z.B. Alterungsschutzmittel, Stabilisatoren, Antiozonantien, Entschäumer, Farbstoffe, anorganische Füllstoffe wie z.B. Kreide, versetzt werden.

Zur Herstellung eines zumindest zweischichtigen Elastomerproduktes wird in der Folge in einem ersten Schritt eine erste Schicht aus einem Elastomer erzeugt, beispielsweise nach einem bekannten Tauchverfahren, und diese vorvernetzt, insbesondere photochemisch vorvernetzt. Gegebenenfalls nach zumindest einem Trocken- und/oder gegebenenfalls zumindest einem Waschschritt wird aus dem modifizierten Latex, der wie voranstehend funktionalisiert wurde, zumindest eine weitere Elastomerschicht auf die zuerst erzeugte Elastomerschicht aufgebracht, insbesondere aufgetaucht. Danach werden wiederum zumindest ein Trocken- und/oder zumindest ein Waschschritt ausgeführt.

Auf die, insbesondere nach einer der beiden Verfahrensvarianten, epoxidierte, d.h. mit Epoxidgruppen versehene Oberfläche des Elastomers werden in der Folge Partikel aufgebracht, wobei diese Partikel über die Epoxidgruppen unter Ringöffnung kovalent an die Elastomeroberfläche gebunden werden.

Insbesondere werden anorganische Feststoffpartikel, vorzugsweise großtechnisch verfügbare Feststoffpartikel, verwendet. Vorzugsweise sind diese Partikel ausgewählt aus einer Gruppe umfassend oder bestehend aus Sulfiden, Oxiden, Hydroxiden, Carbonaten, Boraten, Sulfaten, Phosphaten, Silikaten, Metallpartikel, z.B. Gold, Silber, Kupfer. Insbesondere sind die Feststoffpartikel ausgewählt aus einer Gruppe umfassend oder bestehend aus Kreide, Kieselgur, Kieselerde, Kaolinite, Quarz, amorphe Kieselsäure, $SiO_2$, Calcit, $TiO_2$.

Es können auch Hohlräume aufweisende Partikel, die gegebenenfalls mit einem Wirkstoff beladen sind, beispielsweise Zeolithe oder Cyclodextrine, eingesetzt werden. Gegebenenfalls können diese Partikel auch zur Adsorption von Stoffen, wie z.B. Schweiß, eingesetzt werden.

Es können aber auch organische Feststoffpartikel, beispielsweise zumindest teilweise bestehend aus Stärke oder Cellulose, kovalent an die Elastomeroberfläche angebunden werden.

Von Vorteil ist dabei, wenn die Feststoffpartikel vor der Anbindung an die Epoxigruppen der Elastomeroberfläche oberflächlich funktionalisiert werden. Diese Funktionalisierung kann durch Erzeugung von freien Mercaptogruppen und/oder freien Aminogruppen und/oder Anhydridgruppen und/oder Isocyanatgruppen und/oder Isothiocyanatgruppen und/oder Hydroxygruppen, an der Oberfläche der Feststoffpartikel durchgeführt werden. Insbesondere kann dazu eine chemische Verbindung verwendet werden, die ausgewählt ist aus einer Gruppe umfassend 3-Mercaptopropyltrimethoxysilan, 3-Mercaptopropyltriethoxysilan, 3-Aminopropyltrimethoxysilan, 3-Aminopropyltriethoxysilan, 3-Isocyanatpropyltriethoxysilan, 3-Isocyanatpropyltrimethoxysilan, Hydroxymethyltriethoxysilan. Diese Verbindungen sind jeweils erhältlich bei ABCR bzw. Sigma Aldrich bzw. Evonik Industries.

Es sei in diesem Zusammenhang erwähnt, dass die funktionalisierten Feststoffpartikel zumindest großteils kommerziell erhältlich sind, z.B. bei Hoffmann Mineral bzw. Sigma Aldrich bzw. Evonik Industries.

Die Funktionalisierung der Feststoffpartikel kann aber auch mit zumindest einer chemischen Verbindung durchgeführt werden, die ausgewählt ist aus einer Gruppe umfassend oder bestehend aus Silanen, Siloxanen und Carbonsäuren mit funktionellen Gruppen, wie Anhydrid-, Epoxy-, Isocyanat-, Isothiocyanat-, Mercaptogruppen. Beispiele hierfür sind (3-Glycidoxypropyl)trimethoxysilan, 3-(Triethoxysilyl)propylsuccinic Anhydride, Mercaptopropyltrimethoxysilan. Diese Verbindungen sind erhältlich bei ABCR bzw. Sigma Aldrich.

Die Mercaptogruppe wird insbesondere in Form eines Thiols zur Verfügung gestellt. Bevorzugt werden dazu Thiole verwendet, die ausgewählt sind aus einer Gruppe umfassend oder bestehend aus Trimethylolpropan-tris-3-mercaptopropionat, 16-Mercaptohexadecansäure, (11-Mercaptoundecyl)tetra(ethylenglycol), N-Acetyl-L-cystein, Pentaerythritoltetramercaptoacetat, Trimethylolpropantrimercaptoacetat, Trimethylolpropanetri-3-mercaptopropionat, Pentaerythritoltetra-3-mercaptopropionat, Propylenglycol-3-mercaptopropionat, ethoxyliertes Trimethylolpropantri-3-mercaptopropionat, Polyol-3-mercaptopropionat, Polyester-3-mercaptopropionat. Diese Verbindungen sind beispielsweise bei Bruno Bock Thiochemicals und/oder Sigma Aldrich erhältlich.

Neben diesen bevorzugt verwendeten chemischen Verbindungen mit einer Mercaptogruppe können im Rahmen der Erfindung auch andere derartige Verbindungen eingesetzt werden, wie beispielsweise HS-R1R2R3, wobei R1 durch ein Element aus der Gruppe umfassend oder bestehend aus Alkyl-, Aryl-, Alkylaryl-, Arylalkyl-, Alkylarylalkyl-, Arylalkylaryl-, Silylgruppen, R2 durch ein Element aus der Gruppe umfassend oder bestehend aus Acryl-, Amino-, Aminosäure-, Anhydrid-, Carbonyl- (C=O), Carbonsäure-, Carboxylat,- Epoxy-, Hydroxy-, Isocyanat-, Isothiocyanat-, Methacryl-, Mercapto-, Sulfonsäure-, Vinyl-gruppen, R3 durch ein Element aus der Gruppe umfassend oder bestehend

aus H, Alkyl-, Aryl-gruppen gebildet sind.

Die Aminogruppe wird insbesondere in Form von primären Aminen zur Verfügung gestellt. Bevorzugt werden dazu Amine verwendet, die ausgewählt sind aus einer Gruppe umfassend oder bestehend aus 3-Aminopropyltrimethoxysilan, 3-Aminopropyltriethoxysilan, N-2(aminoethyl)3-aminopropyltriethoxysilan.

Im Rahmen der Erfindung können aber auch nicht funktionalisierte Partikel eingesetzt werden, insbesondere wenn diese aufgrund des chemischen Aufbaus bereits mit funktionellen Gruppen versehen sind.

Zur Funktionalisierung wird das zumindest eine Thiol und/oder Amin und/oder Anhydrid und/oder Isocyanat und/oder Isothiocyanat und/oder die Verbindung mit den Hydroxygruppen in einem Lösungsmittel, insbesondere Wasser, wobei auch organische Lösungsmittel verwendet werden können, gelöst. Die Konzentration an dem zumindest einen Thiol und/oder Amin und/oder Anhydrid und/oder Isocyanat und/oder Isothiocyanat und/oder die Verbindung mit den Hydroxygruppen und/oder mit der zumindest einen chemischen Verbindung, die ausgewählt ist aus der Gruppe umfassend oder bestehend aus Silanen, Siloxanen und Carbonsäuren mit funktionellen Gruppen, wie Anhydrid-, Epoxy-, Isocyanat-, Isothiocyanat-, Mercaptogruppen kann zwischen 0,1 Gew.-% und 50 Gew.-%, insbesondere zwischen 0,5 Gew.-% und 15 Gew.-%, jeweils bezogen auf das Gesamtgewicht mit den (anorganischen) Partikeln, betragen.

In diese Lösung werden in der Folge die jeweiligen Partikel zur Funktionalisierung gegeben.

Die Funktionalisierung der Feststoffpartikel kann bei einer Temperatur zwischen 0 °C und 200 °C und/oder für einer Dauer zwischen 5 Minuten und 720 Minuten erfolgen und/oder bei eine pH-Wert zwischen 3 und 6. Vorzugsweise findet die Funktionalisierung unter Rühren statt.

Die Umsetzung selbst kann sowohl in wässrigen Medien als auch in flüssigen organischen Medien, beispielsweise Ethanol, Toluol, Cyclohexan, Hexan, Isopropanol, erfolgen. Ein möglicher schematischer Verfahrensablauf ist in Fig. 1 dargestellt, wobei als Partikel aminofunktionalisierte $SiO_2$-Partikel verwendet werden.

Durch diese Vorgangsweise wird der Vorteil erreicht, dass ein quantitatives Entfernen von nicht kovalent gebundenen Partikeln möglich ist, da die Klebrigkeit der Elastomeroberfläche im ersten Schritt durch die Absättigung der ungesättigten Kohlenstoff-Kohlenstoff Bindungen deutlich verringert wird.

Die funktionalisierten Partikel werden in Wasser oder einem organischen Lösungsmittel suspendiert. Die Suspension wird vorzugsweise mit einer Konzentration zwischen 0,01 Gew.-% und 10 Gew.-%, insbesondere zwischen 0,01 Gew.-% und 1 Gew.-%, an mit Aminogruppe(n)- und/oder Mercaptogruppe(n)- und/oder Carbonsäuregruppe(n) und/oder Epoxidgruppe(n) und/oder Hydroxygruppe(n) und/oder Anhydridgruppe(n) und/oder Isocyanatgruppe(n) und/oder Isothiocyanatgruppe(n) -modifizierten Partikel mittels handelsüblicher Dispergiergeräte hergestellt. Danach wird diese Suspension mit der funktionalisierten Elastomeroberfläche in Kontakt gebracht, beispielsweise durch Eintauchen des Elastomers in die Suspension. Gegebenenfalls kann dies mehrmals durchgeführt werden. Es ist dabei auch möglich, dass das Elastomer nur teilweise eingetaucht wird. Bei einem mehrmaligen Tauchen dieses teilweise Eintauchen auf ein oder mehrere Tauchschritte beschränkt sein, wobei es auch hier möglich ist, dass bei sämtlichen Tauchschritten nur teilweise eingetaucht wird.

Anschließend wird das so behandelte Elastomer getrocknet. Die Temperatur kann dabei ausgewählt sein aus einem Bereich von 40 °C bis 150 °C, insbesondere aus einem Bereich von 40 °C bis 100 °C. Die Trocknung kann während einer Zeitspanne zwischen 5 Minuten und 1000 Minuten, insbesondere während einer Zeitspanne zwischen 10 Minuten und 900 Minuten, erfolgen.

Während der Trocknung erfolgt die kovalente Anbindung der Feststoffpartikel an die Elastomeroberfläche.

Bevorzugt werden nach der Anbindung rein adhäsiv gebundene Partikel von der Oberfläche des Elastomerproduktes entfernt, beispielsweise durch Waschen und/oder mechanisch, beispielsweise mittels Ultraschall.

Die im Rahmen der Erfindung verwendeten Feststoffpartikel haben vorzugsweise eine Partikelgröße zwischen 10 nm und 10 $\mu$m, insbesondere zwischen 20 nm und 50 nm bzw. zwischen 1 $\mu$m und 5 $\mu$m.

Prinzipiell besteht die Möglichkeit, dass die gesamte Oberfläche des epoxidierten Elastomers mit zumindest einer Art von Partikeln - es können auch zumindest zwei unterschiedliche Arten von Partikeln, beispielsweise sowohl Quarz als auch Zeolith, eingesetzt werden, um damit ein anderes Eigenschaftsprofil des Elastomers zu erreichen - versehen wird.

Gemäß einer Ausführungsvariante dazu kann jedoch vorgesehen sein, dass die Partikel lediglich in diskreten Bereichen an der Elastomeroberfläche angeordnet werden. Um dies zu erreichen, kann mit einer entsprechenden Maske der nicht weiter zu funktionalisierende Bereich der Elastomeroberfläche abgedeckt werden, sodass in einen anschließenden Waschschritt die nicht kovalent gebundenen Partikel abgewaschen werden.

Nach einer Ausführungsvariante dazu besteht die Möglichkeit, dass die Partikel bereits nur in diskreten Bereichen aufgetragen werden.

Die Maske kann eine mechanische Maske oder eine chemische Maske oder eine optische Maske sein. Unter einer chemischen Maske wird eine Substanz verstanden, die vor dem Auftragen der jeweiligen Suspensionen oder Emulsionen oder Lösung auf die nicht zu beschichtenden Bereiche aufgebracht wird, beispielsweise aufgestrichen.

Ebenso ist es möglich, dass die Epoxidierung des Elastomers nur in den mit den Partikel zu versehenden Bereichen durchgeführt wird, wozu ebenfalls entsprechende Masken, wie ausgeführt, eingesetzt werden können.

Neben der reinen Oberflächenstrukturierung kann damit beispielsweise auch eine dauerhafte Anbringung von Informationen auf dem Elastomerprodukt, beispielsweise der Handschuhgröße bei Elastomerhandschuhen, erreicht werden.

Nach einer weiteren Ausführungsvariante kann vorgesehen werden, dass die Partikel nach der kovalenten Anbindung an die Elastomeroberfläche miteinander zumindest teilweise vernetzt werden.

Diese Vernetzung kann über nicht reagierte funktionelle Gruppen der voranstehend beschriebenen funktionalisierten Partikel erreicht werden, wobei gegebenenfalls ein zusätzliches Reagens verwendet wird, dass mit diesen funktionellen Gruppen reagiert und damit eine Verbindung zwischen den Partikel herstellt.

Es ist aber auch der Einsatz von mehrfach funktionellen Thiolen und/oder Aminen oder und/oder Anhydriden und/oder Isocyanaten und/oder Isothiocyanaten, d.h. von chemischen Verbindungen mit mehr als einer Mercapto- und/oder Amino- oder Carbonsäuregruppe und/oder Epoxidgruppe und/oder Hydroxygruppe und/oder Anhydridgruppe und/oder Isocyanatgruppe und/oder Isothiocyanatgruppe, wie beispielsweise Trimethylolpropan-tris-3-mercaptopropionat, möglich, womit freie Mercaptogruppen zumindest an der Oberfläche des zuvor epoxidierten Elastomers erzeugt werden können, über die eine weitere Reaktion mit weiteren chemischen Verbindungen zur weiteren Veränderung der Eigenschaften des Elastomerproduktes ermöglicht wird.

Neben der Ausführungsvariante mit chemischen Verbindungen mit mehrfacher Homofunktionalität, also mit Verbindungen die ausschließlich Mercaptogruppen als funktionelle Gruppen im Molekül aufweisen, besteht auch die Möglichkeit, mehrfach funktionelle chemische Verbindung mit Heterofunktionalität einzusetzen. Bei diesen Verbindungen ist beispielsweise neben zumindest einer Mercaptogruppe, über die die Anbindung der Verbindung an die Partikeloberfläche erfolgt, zumindest eine weitere Funktionalität vorhanden, beispielsweise eine Aminogruppe, eine Carbonsäuregruppe, eine Epoxidgruppe, eine Hydroxygruppe, eine Anhydridgruppe, eine Isocyanatgruppe, eine Isothiocyanatgruppe, eine Vinylgruppe, wobei auch Mischvarianten möglich sind, sodass also mehr als eine dieser Gruppen zusätzlich zu der oder den Mercaptogruppen vorhanden sind, beispielsweise eine Carbonsäuregruppe und eine Aminogruppe.

Bei Verwendung von mehrfach funktionellen Thiolen oder mehrfach funktionellen Aminen oder mehrfach funktionellen Polycarbonsäuren, Polyalkoholen, Polyacetalen können damit auch reaktive Gruppen an der Oberfläche der Partikel erzeugt werden, beispielsweise weitere Thiolgruppen oder Aminogruppen oder Carbonsäuregruppen.

Es können auch Polymere mit funktionellen Gruppen (Alkene, Acrylate, Anhydride, Epoxide, Isocyanate, Isothiocyanate, Methacrylate, Thiole) zur Modifizierung der Partikel verwendet werden um eine kovalente Bindung an die Elastomeroberfläche zu erzielen. Die Polymere können in Dispersion, in Lösung und als Reinsubstanz eingesetzt werden.

Die funktionellen Gruppen können als Seitengruppen oder endständig vorliegen.

Die reaktiven Gruppen können dazu verwendet werden, um die funktionalisierten Partikel mit weiteren chemischen Verbindungen zu versehen, die mit diesen Gruppen reagieren können.

Die weitere chemische Verbindung kann ausgewählt werden aus einer Gruppe umfassend Acrylate, Amine, Aminosäuren (Cystein), acetylierte Aminosäuren (N-Acetylcystein), Anhydride, Carbonsäuren, Ether, Epoxide, Isocyanate, Isothiocyanate, Methacrylate, Silane, Siloxane. Es wird damit eine zusätzliche Verbesserung der Gleitreibungseigenschaften erreicht bzw. kann eine andere Funktionalität (für Folgereaktionen) eingebracht werden.

Diese Umsetzung kann je nach Reaktionspartner bei einer Temperatur zwischen 20 °C und 80 °C und nach bekannten Reaktionsmechanismen erfolgen. Die Dauer dieser Umsetzung richtet sich ebenfalls nach den jeweiligen konkreten Verbindungen die zur Reaktion gebracht werden und kann zwischen 1 Minute und 100 Minuten betragen. Gegebenenfalls kann die Umsetzung unter Druck oder unter Vakuum durchgeführt werden.

Nach einer anderen Ausführungsvariante der Erfindung ist vorgesehen, dass die funktionellen Gruppen auf der Partikeloberfläche, die nicht zur kovalenten Anbindung an das Elastomer benötigt werden, zumindest bereichsweise mit einer Polymerschicht kovalent verbunden werden.

Die Polymerschicht kann beispielsweise aus einem Polyurethan oder einem Silikon oder aus einer Mischung aus SBR mit Silikon oder einem Acrylat oder einem Siloxan oder aus einem Polymer mit funktionellen Gruppen, insbesondere Alkene, Acrylate, Anhydride, Epoxide, Isocyanate, Isothiocyanate, Methacrylate, Thiole, Alkohole, Carbonsäuren, hergestellt werden. Gegebenenfalls können die Polymere bzw. Monomere zur Bildung der Polymerschicht ebenfalls vorher funktionalisiert werden, insbesondere mit zumindest einer Art der erwähnten funktionellen Gruppen.

Bevorzugte Polymere sind Silikone, Polyurethane, Urethanacrylate, Acrylate, Polyisocyanate, Polyester Polyole, Vinylpolymere, Dien-Elastomere. Beispiele hierfür sind Desmophen® 1652, Synthomer VL 11005, Desmolux® XP 2740, Bayhydrol® UV XP 264, Desmolux® VP LS 2299, Polyvinylalkohol, Polyacrylsäure, beziehbar bei Bayer bzw.Synthomer.

Aus den gegebenenfalls funktionalisierten Polymeren oder Monomeren bzw. Oligomeren werden wiederum Suspensionen erzeugt (zur Funktionalisierung kann das jeweilige Reagens dieser Suspension zugesetzt werden), wobei zumindest ein Emulgator und/oder zumindest ein Photoinitiator, falls die Monomere oder Oligomere photochemisch vernetzt werden, zugesetzt werden können.

Die Partikel können auch dazu verwendet werden, um darauf und/oder darin zumindest einen Wirkstoff vorzusehen. Mögliche Wirkstoffe sind z.B. Antitranspiranzien, antibakterielle Wirkstoffe, Fungizide, Geruchsstoffe, Hautpflegemittel, wie z.B. ein Vitamin oder Aloe Vera, Pigmente, Wirkstoffe zur Veränderung des Wasseraufnahmeverhaltens, Stabilisatoren, etc..

Es wird mit dem Verfahren nach der Erfindung eine Funktionalisierung der Funktionalisierung, d.h. der funktionalisierten Elastomeroberfläche, durchgeführt. Die infolge der ersten Funktionalisierung auf der Oberfläche angeordneten funktionellen Gruppen wirken dabei als Ankergruppen für die weitere Funktionalisierung.

Mit dem Verfahren nach der Erfindung können Elastomerprodukte hergestellt werden, die eine bessere Gleitfähigkeit und eine bessere Alterungsbeständigkeit im Vergleich zu einem unbehandelten Elastomer aufweisen. Darüber hinaus können damit Eigenschaften, wie z.B. eine hautpflegende Wirkung, das Wasseraufnahmeverhalten, etc. beeinflusst werden bzw. dem Elastomerprodukt völlig neue Eigenschaften verliehen werden, wie z.B. strukturierte Elastomeroberflächen, Geruch, Farbe, "Look and Feel".

Es wurde mit Hilfe von tribologischen Messmethoden mit einem Lineartribometer entsprechend B. Bhushan, Modern tribology handbook. CLC-Press, Boca Raton, London , New York, Washington D.C. 2001, der Gleitreibungskoeffizient von Partikel modifizierten NR-Oberflächen bestimmt und mit den Eigenschaften von kommerziellen Operationshandschuhen verglichen. Die Ergebnisse in folgender Tabelle zeigen, dass die Gleitreibungseigenschaften von mit Partikel modifizierten Elastomeroberflächen im Bereich von gepuderten NR-Oberflächen liegen.

Vergleich der Gleitreibungskoeffizienten von ausgewählten NR-Oberflächen

| Probenbeschreibung | Gleitreibungskoeffizient m |
|---|---|
| Stand der Technik Handschuh mit chlorierter Innenseite | m ~ 0,31 |
| Stand der Technik Handschuh mit beschichteter Innenseite | m ~ 0,22 |
| Stand der Technik Handschuh mit gepuderter Innenseite | m ~ 0,50 |
| Mit aminofunktionalisierten $SiO_2$-Partikel modifizierte NR-Oberfläche | m ~ 0,7 |

Im Folgenden sind einige im Zuge der Erarbeitung der Erfindung durchgeführte, nicht beschränkend zu verstehende Beispiele angegeben.
Die für die Beispiele verwendeten Chemikalien sind in Tabelle 1 zusammengestellt.

**Tabelle 1: verwendete Materialien und Chemikalien**

| Chemikalie | Hersteller | Strukturformel, Spezifikation |
|---|---|---|
| Aktisil AM | Hoffmann Mineral | Amino-modifizierte $SiO_2$-Partikel ($d_{50}$ = 2,2 μm) |
| Aktisil MM | Hoffmann Mineral | Mercapto-modifizierte $SiO_2$-Partikel ($d_{50}$ = 2,2 μm) |
| Nanopartikel | Sigma Aldrich | Amino-modifizierte $SiO_2$-Partikel ($d_{50}$= 20 nm) |
| Peressigsäure konz. (39 %-ig) | Sigma Aldrich | |
| Ameisensäure | Sigma Aldrich | |
| $H_2O_2$ | Sigma Aldrich | |
| Synperonic NP 30 | Sigma Aldrich | 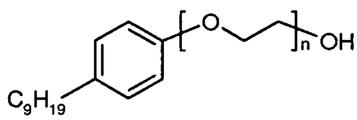 |

1. Modifizierung der getrockneten Filmoberfläche

1.1 Epoxidierung mit Persäuren

Bei der Epoxidierung von NR-Filmen wurden nachfolgende Schritte durchlaufen:

- Waschen des getrockneten Latexfilms in deionisiertem Wasser (10 min bei RT (= Raumtemperatur, = 20 °C))
- Epoxidieren des gewaschenen Latexfilms in einer wässrigen Peressigsäurelösung (2 Gew.-%) bei 40°C für 40 min
- Kurzes Eintauchen des epoxidierten Latexfilms in deionisiertem Wasser (1 min bei RT)
- Trocknen bei 70°C für 15 min.

1.2 Epoxidierung der flüssigen Phase

1.2.1 Epoxidierung mit Persäuren

Bei der Epoxidierung des flüssigen NR-Latex wurden nachfolgende Schritte durchlaufen:

- Stabilisierung des NR-Latex mit Synperonic NP 30 (10 phr) und Einstellung des Feststoffgehalts von 20 - 60 Gew.-%
- Latex wird mit Essigsäure auf einen pH Wert von 3 - 4 angesäuert
- Wahlweise Zugabe von Peressigsäure (39%ige Lösung), molares Konzentrationsverhältnis beträgt:

$$\frac{[Persäure]}{[Polyisopreneinheit]} = 0{,}07 - 0{,}5$$

bzw. 0,07 Mol-% bis 0,5 Mol-% Peressigsäure bezogen auf die Isopreneinheiten oder in-situ Bildung von Perameisensäure durch $H_2O_2$ und Ameisensäure mit einem molaren Konzentrationsverhältnis von:

$$\frac{[H_2O_2]}{[Polyisopreneinheit]} = \frac{[HCOOH]}{[Polyisopreneinheit]} = 0{,}2 - 1{,}0$$

- Reaktionstemperatur 21 - 60°C
- Reaktionszeit bis 3 - 48 h
- Beenden der Reaktion durch Neutralisation mit einer KOH-Lösung (10 Gew.-%)

Beispielsweise können 4g Synperonic in 33,2g H2O bei 40°C gelöst und zu 67g NR(60%drc) zugegeben werden. Dieser Ansatz wird für 15 Stunden gerührt. Danach wird auf pH~4 angesäuert und werden 28,44g HCOOH(98-100%) und 33,26g $H_2O_2$ (30-%) langsam zugegeben.

2. Herstellung von Filmen aus epoxidiertem NR-Latex

Die entsprechenden Latexfilme werden in einer Zweischichttauchung hergestellt:

- Auftauchen eines vorvernetzten NR-Latex auf eine Porzellanform (20s bei RT)
- 0 - 15 s trocknen bei 120°C
- Auftauchen des epoxidierten NR-Latex (30s bei RT)
- Trocknen für 20 min bei 120°C
- Abziehen des Films

3. Ankopplung von anorganischen Partikeln

Beispiel 1: Durchführung in wässrigen Systemen

Im Zuge der Ankopplung von anorganischen $SiO_2$-Makropartikeln wurden nachfolgende Prozessschritte durchgeführt:

- Herstellen einer wässrigen Suspension mit 0,015 - 0,5 Gew.-% kommerziell erhältlicher Amino- bzw. Mercapto-modifizierter $SiO_2$-Makropartikel durch Einwaage der Partikel in $H_2O$ deionisiert
- Dispergieren der Suspension mit Dispergiergerät (Ultraturax) für 10 min bei Raumtemperatur und anschließend im Ultraschallbad für 10 min bei Raumtemperatur
- Fixieren eines epoxidierten NR-Latexfilms in einer Petrischale
- Übergießen des Elastomerfilms mit der wässrigen Suspension
- Film nach 2 min aus der Petrischale entnehmen
- Trocknen der Probe für 10 - 900 min bei 40 - 100°C
- Film für 16 h bei Raumtemperatur in Wasser waschen
- Film für 10 - 15 min bei 70 °C trocknen

Beispiel 2: Durchführung in organischen Lösungsmitteln

- Herstellen einer Suspension mit 0,015 - 0,2 Gew.-% Amino- bzw. Mercapto- modifizierter $SiO_2$-Makropartikel in Toluol
- Dispergieren der Suspension im Ultraschallbad für 10 min bei Raumtemperatur
- Übergießen des Elastomerfilms mit der Suspension, Verhindern des Aufschwimmens des Films mithilfe einer Pinzette
- Film nach 2 min aus der Petrischale entnehmen
- Trocknen der Probe für 10 - 900 min bei 40 - 100°C
- Film für 16 h bei Raumtemperatur in Toluol waschen
- Film für 10 - 15 min bei 70 °C trocknen

Beispiel 3: Ankopplung von nanoskalierten Partikeln

- Herstellen einer Suspension mit 0,015 - 0,05 Gew.-% Amino - modifizierter $SiO_2$-Nanopartikel (suspendiert in Ethanol) in Toluol
- Dispergieren der Suspension im Ultraschallbad für 30 min bei Raumtemperatur
- Übergießen des Elastomerfilms mit der Suspension, verhindern des Aufschwimmens des Films mithilfe einer Pinzette
- Film nach 2 min aus der Petrischale entnehmen
- Trocknen der Probe für 10 - 900 min bei 40 - 100°C
- Film für 16 h bei Raumtemperatur in Toluol waschen
- Film für 10 - 15 min bei 70 °C trocknen

Großtechnisch kann die Umsetzung des Verfahrens beispielsweise direkt an einer Kettentauchanlage durch Eintauchen der Handschuhe auf der Form in die Partikeldispersion erfolgen. Dazu ist es von Vorteil, wenn die Partikel in dem entsprechenden Tauchbecken zumindest zeitweise, bevorzugt ständig, dispergiert werden und die verbrauchten Partikel nachdosiert werden. Die thermische Ankopplung kann während der Trocknung bzw. wahlweise Vulkanisation (bei S-vulkanisierten Handschuhen) der Handschuhe im Ofen erfolgen. Anschließend können die Elastomerprodukte mit Wasser gewaschen werden, um nicht gebundene Partikel zu entfernen.

Alternativ kann die Aufbringung der Partikel auch über das Eintauchen der Handschuhe (auf der Form) in Partikelslurrys erfolgen.

## Patentansprüche

1. Verfahren zur Anbindung von anorganischen oder organischen Feststoffpartikeln an die Oberfläche eines Elastomerhandschuhs, wobei das Elastomer in der Molekülstruktur ungesättigte Kohlenstoff-Kohlenstoff Bindungen aufweist, die zumindest im Bereich der Oberfläche des Elastomerhandschuhs zumindest teilweise durch Epoxidierung abgesättigt werden, **dadurch gekennzeichnet, dass** die Feststoffpartikel nach der Epoxidierung der Elastomeroberfläche an die Epoxidgruppen kovalent angebunden werden.

2. Verfahren nach Anspruch 1, **dadurch gekennzeichnet, dass** die Feststoffpartikel vor der Reaktion oberflächlich funktionalisiert werden.

3. Verfahren nach Anspruch 2, **dadurch gekennzeichnet, dass** die Funktionalisierung der Feststoffpartikel durch Erzeugung von freien Mercaptogruppen und/oder freien Aminogruppen und/oder Carbonsäuregruppen und/oder Epoxidgruppen und/oder Hydroxygruppen und/oder Anhydridgruppen und/oder Isocyanatgruppen und/oder Isothiocyanatgruppen, an der Oberfläche der Feststoffpartikel durchgeführt wird.

4. Verfahren nach Anspruch 2 oder 3, **dadurch gekennzeichnet, dass** die Funktionalisierung der Feststoffpartikel mit zumindest einer chemischen Verbindung durchgeführt wird, die ausgewählt ist aus einer Gruppe umfassend 3-Aminopropyltrimethoxysilan, 3-Mercaptopropyltriethoxysilan, Hydroxymethyltriethoxysilan, 3-Isocyanatpropyltrimethoxysilan, 3-Glycidoxypropyltrimethoxysilan, 3-(Triethoxysilyl)propylsuccinic Anhydrid.

5. Verfahren nach einem der Ansprüche 1 bis 4, **dadurch gekennzeichnet, dass** adhäsiv gebundene Feststoffpartikel

von der Oberfläche des Elastomers entfernt werden.

6. Verfahren nach einem der Ansprüche 1 bis 5, **dadurch gekennzeichnet, dass** die Epoxidierung des Elastomers an einer festen Oberfläche ausgeführt wird.

7. Verfahren nach einem der Ansprüche 1 bis 6, **dadurch gekennzeichnet, dass** die Epoxidierung an einem Latex in flüssiger Phase durchgeführt wird.

8. Verfahren nach einem der Ansprüche 1 bis 8, **dadurch gekennzeichnet, dass** die Epoxidierung in diskreten Bereichen des Elastomers durchgeführt wird.

9. Elastomerhandschuh aus einem Elastomer mit einer Oberfläche, wobei das Elastomer in der Molekülstruktur ungesättigte Kohlenstoff-Kohlenstoff Bindungen aufweist, **dadurch gekennzeichnet, dass** die Oberfläche zumindest teilweise kovalent an das Elastomer gebundene anorganische oder organische Feststoffpartikel aufweist.

10. Elastomerhandschuh nach Anspruch 9, **dadurch gekennzeichnet, dass** dieses zumindest zweischichtig ausgebildet ist.

11. Elastomerhandschuh nach Anspruch 9 oder 10, **dadurch gekennzeichnet, dass** die Feststoffpartikel nur in diskreten Bereichen der Oberfläche angeordnet sind.

12. Elastomerhandschuh nach Anspruch einem der Ansprüche 9 bis 11, **dadurch gekennzeichnet, dass** die Feststoffpartikel einen Partikeldurchmesser zwischen 10 nm und 10 μm aufweisen.

13. Elastomerhandschuh nach einem der Ansprüche 9 bis 12, **dadurch gekennzeichnet, dass** die Feststoffpartikel miteinander zumindest teilweise vernetzt sind.

14. Elastomerhandschuh nach einem der Ansprüche 9 bis 14, **dadurch gekennzeichnet, dass** die Feststoffpartikel zumindest einen Wirkstoff aufweisen.

**Claims**

1. A method of bonding inorganic or organic solid particles to the surface of an elastomer glove, the elastomer in the molecular structure comprising unsaturated carbon-carbon bonds which are at least partially saturated by epoxidation at least in the region of the surface of the elastomer glove, **characterized in that** the solid particles are covalently bonded to the epoxide groups after epoxidation of the elastomer surface.

2. The method according to claim 1, **characterized in that** the surface of the solid particles is functionalized prior to the reaction.

3. The method according to claim 2, **characterized in that** the functionalization of the solid particles is effected by creating free mercapto groups and/or free amino groups and/or carboxylic acid groups and/or epoxide groups and/or hydroxy groups and/or anhydride groups and/or isocyanate groups and/or isothiocyanate groups on the surface of the solid particles.

4. The method according to claim 2 or 3, **characterized in that** the solid particles are functionalized by means of at least one chemical compound selected from a group comprising 3-aminopropyltrimethoxysilane, 3-mercaptopropyltriethoxysilane, hydroxymethyltriethoxysilane, 3-isocyanate propyltrimethoxysilane, 3-glycidoxypropyltumethoxysilane, 3-(triethoxysilyl)propylsuccinic anhydride.

5. The method according to one of claims 1 to 4, **characterized in that** adhesively bonded solid particles are removed from the surface of the elastomer.

6. The method according to one of claims 1 to 5, **characterized in that** the epoxidation of the elastomer is effected on a solid surface.

7. The method according to one of claims 1 to 6, **characterized in that** epoxidation is effected on a latex in liquid phase.

8. The method according to one of claims 1 to 8, **characterized in that** epoxidation is effected in discrete regions of the elastomer.

9. An elastomer glove made of elastomer with a surface, the elastomer in the molecular structure comprising unsaturated carbon-carbon bonds, **characterized in that** the surface has inorganic or organic solid particles at least partially covalently bonded to the elastomer.

10. The elastomer glove according to claim 9, wherein it comprises at least two layers.

11. The elastomer glove according to claim 9 or 10, **characterized in that** the solid particles are disposed only in discrete regions of the surface.

12. The elastomer glove according to one of claims 9 to 11, **characterized in that** the solid particles have a particle diameter of between 10 nm and 10 $\mu$m.

13. The elastomer glove according to one of claims 9 to 12, wherein the solid particles are at least partially cross-linked with one another.

14. The elastomer glove according to one of claims 9 to 14, **characterized in that** the solid particles incorporate at least one active substance.

**Revendications**

1. Procédé pour lier des particules solides inorganiques ou organiques à la surface d'un gant en élastomère, l'élastomère présentant dans sa structure moléculaire des liaisons carbone-carbone insaturées, qui au moins dans la zone de la surface du gant en élastomère sont au moins partiellement saturées par époxydation, **caractérisé en ce que** les particules solides sont, après l'époxydation de la surface élastomère, liées par liaison covalente aux groupes époxydes.

2. Procédé selon la revendication 1, **caractérisé en ce que** les particules solides sont fonctionnalisées en surface avant la réaction.

3. Procédé selon la revendication 2, **caractérisé en ce que** la fonctionnalisation des particules solides est mise en oeuvre par production de groupes mercapto libres et/ou de groupes amino libres et/ou de groupes acide carboxylique et/ou de groupes époxydes et/ou de groupes hydroxy et/ou de groupes anhydride et/ou de groupes isocyanate et/ou de groupes isothiocyanate, sur la surface des particules solides.

4. Procédé selon la revendication 2 ou 3, **caractérisé en ce que** la fonctionnalisation des particules solides est mise en oeuvre avec au moins un composé chimique qui est choisi dans le groupe consistant en le 3-aminopropyltriméthoxysilane, le 3-mercaptopropyl-triéthoxysilane, l'hydroxyméthyltriéthoxysilane, le 3-isocyanatopropyl-triméthoxysilane, le 3-glycidoxypropyl-triméthoxysilane, l'anhydride 3-(triéthoxy-silyl)propylsuccinique.

5. Procédé selon l'une des revendications 1 à 4, **caractérisé en ce que** des particules solides liées par adhérence sont éliminées de la surface de l'élastomère.

6. Procédé selon l'une des revendications 1 à 5, **caractérisé en ce que** l'époxydation de l'élastomère est réalisée sur une surface solide.

7. Procédé selon l'une des revendications 1 à 6, **caractérisé en ce que** l'époxydation est mise en oeuvre en phase liquide sur un latex.

8. Procédé selon l'une des revendications 1 à 8, **caractérisé en ce que** l'époxydation est mise en oeuvre dans des zones discrètes de l'élastomère.

9. Gant en élastomère, obtenu à partir d'un élastomère ayant une surface, l'élastomère présentant dans sa structure moléculaire des liaisons carbone-carbone insaturées, **caractérisé en ce que** la surface présente au moins partiellement des particules solides inorganiques ou organiques liées par liaison covalente à l'élastomère.

**10.** Gant en élastomère selon la revendication 9, **caractérisé en ce que** ce gant est réalisé en au moins deux couches.

**11.** Gant en élastomère selon la revendication 9 ou 10, **caractérisé en ce que** les particules solides ne sont disposées que dans des zones discrètes de la surface.

**12.** Gant en élastomère selon l'une des revendications 9 à 11, **caractérisé en ce que** les particules solides présentent une granulométrie comprise entre 10 nm et 10 μm.

**13.** Gant en élastomère selon l'une des revendications 9 à 12, **caractérisé en ce que** les particules solides sont au moins partiellement réticulées les unes avec les autres.

**14.** Gant en élastomère selon l'une des revendications 9 à 14, **caractérisé en ce que** les particules solides comprennent au moins une matière active.

Fig.1

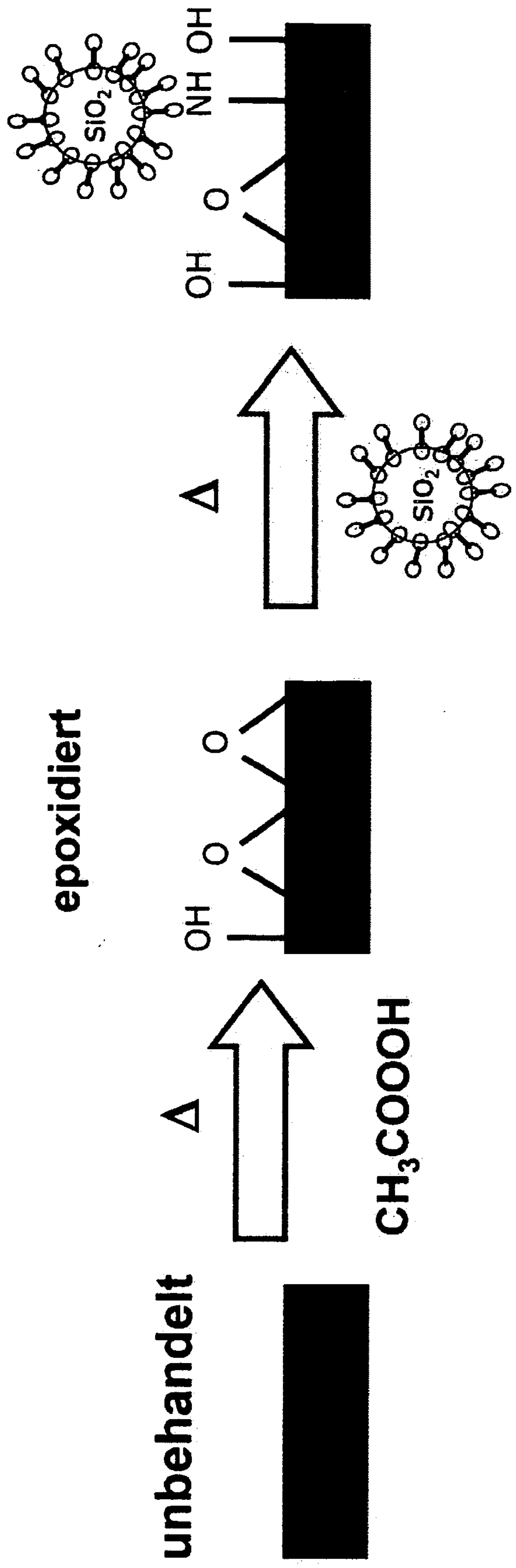

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 7442746 A
- DE 10260219 B
- GB 1396090 A
- US 5310819 A
- US 5804318 A
- US 6797783 A
- US 2006074185 A1
- DD 296853 A5
- AT 502764 A1
- AT 508099 A1


### In der Beschreibung aufgeführte Nicht-Patentliteratur


- Modification of epoxidised natural rubber film surface by polymerisation of methyl methacrylate. **AMORN-CHAIYAPITAK C. et al.** European Polymer Journal. Pergamon Press Ltd, 01. Juni 2008, vol. 44, 1782-1788
- **THONGNUANCHAN B. et al.** Epoxidized natural nubber-bonded wood Particleboard. *Polymer Engineering & Science,* 01. Marz 2007, vol. 47 (4), 421-428
- **B. BHUSHAN.** Modern tribology handbook. CLC-Press, 2001